# EUROPÄISCHE PATENTSCHRIFT

(11) **EP 2 919 013 B1**
(45) Veröffentlichungstag und Bekanntmachung des Hinweises auf die Patenterteilung: **02.11.2016**
(21) Anmeldenummer: 14158716.2
(22) Anmeldetag: 11.03.2014
(51) Int. Cl.: G01N 33/86

(54) **Verfahren zur Detektion von Modulatoren der GPIb-Thrombin-Interaktion**
Method for the detection of modulators of GPIb thrombin interaction
Procédé de détection de modulateurs de l'interaction GPIb-Thrombine

(43) Veröffentlichungstag der Anmeldung: 16.09.2015
(73) Patentinhaber: Siemens Healthcare Diagnostics Products GmbH, 35041 Marburg (DE)
(72) Erfinder: Patzke, Juergen, 35043 Marburg (DE)

(56) Entgegenhaltungen:
- EP-A1- 2 637 024
- WO-A1-2009/026551
- DE-A1-102007 031 708
- Pozzi Nicola: "STruttura e Funzione dei FAttori della Coagulazione", , 21. Januar 2010 (2010-01-21), Seiten 1-202, XP002728597, Universita degli studi di Padova Gefunden im Internet: URL:http://paduaresearch.cab.unipd.it/2395 /1/Ph.D._Thesis_-_Nicola_Pozzi_21-01-2010. pdf [gefunden am 2014-08-13]
- SHEN YANG ET AL: "Functional analysis of the C-terminal flanking sequence of platelet glycoprotein Ibalpha using canine-human chimeras", BLOOD, Bd. 99, Nr. 1, 1. Januar 2002 (2002-01-01) , Seiten 145-150, XP002728598, ISSN: 0006-4971
- OTHMAN MAHA ET AL: "Platelet-Type von Willebrand Disease: New Insights into the Molecular Pathophysiology of a Unique Platelet Defect", SEMINARS IN THROMBOSIS AND HEMOSTASIS, Bd. 39, Nr. 6, September 2013 (2013-09), Seiten 663-673, XP002728599,

## Beschreibung

Die vorliegende Erfindung liegt auf dem Gebiet der Gerinnungsdiagnostik und betrifft Verfahren zur Detektion von Modulatoren der GPIb-Thrombin-Interaktion in einer Probe.

Thrombin (Faktor IIa) ist an einer Vielzahl von aktivierenden und inhibierenden Mechanismen der plasmatischen Blutgerinnung beteiligt und ist damit das zentrale Enzym der sekundären Hämostase, die in erster Linie die Fibrinbildung umfasst. Weniger gut untersucht und verstanden ist die Rolle von Thrombin in der primären Hämostase, die die Plättchenaktivierung und Plättchenadhäsion infolge einer Endothelverletzung umfasst. Es ist bekannt, dass Thrombin ein Plättchenaktivator ist und die Plättchenaggregation stimuliert. Die wichtigsten Thrombin-Rezeptoren auf der Plättchenoberfläche sind die PAR-Rezeptoren (protease activated receptors) einerseits und der Glykoprotein Ib-V-IX-Rezeptorkomplex andererseits. Der Glykoprotein Ib-V-IX-Rezeptorkomplex umfasst das integrale Membranprotein Glykoprotein Ib (GPIb), das integrale Membranprotein Glykoprotein IX (GPIX) und Glykoprotein V (De Candia, E., Mechanisms of platelet activation by thrombin: A short history. Thrombosis Research 2012, 129: 250-256).

GPIb ist ein zweikettiges Molekül, das aus einer schweren Kette mit einer apparenten molekularen Masse von etwa 145 kDa (synonym: heavy chain, alpha-Kette oder GPIbα) und einer leichten Kette mit einer apparenten molekularen Masse von etwa 22 kDa (synonym: light chain, beta-Kette oder GPIbβ) besteht, welche über Disulfidbrücken miteinander verbunden sind (Lopez, J.A. et al., Cloning of the α chain of human platelet glycoprotein Ib: A transmembrane protein with homology to leucine-rich α2-glycoprotein. Proc. Natl. Acad. Sci USA 1987, 84: 5615-5619).

Das GPIbα-Protein enthält Bindungsstellen für das Thrombin und bewirkt damit die Bindung von Thrombin an den Glykoprotein Ib-V-IX-Rezeptorkomplex. Glycocalicin ist ein Fragment der GPIbα-Kette, das proteolytisch von dem intakten Rezeptor in der Plättchenmembran abgespalten wird. Glycocalicin ist im Plasma nachweisbar. Erhöhte Konzentrationen von freiem Glycocalicin im Plasma weisen auf eine Störung der Plättchenfunktion hin (Beer, J.H. et al., Glycocalicin: A New Assay - The Normal Plasma Levels And Its Potential Usefulness in Selected Diseases. Blood 1994, 83(3): 691-702).

Da Thrombin und Plättchen eine zentrale Rolle bei der Entstehung arterieller Thrombosen spielen und mittlerweile Inhibitoren der Plättchenaggregation zur prophylaktischen und therapeutischen Anwendung erforscht und angewendet werden, ist die gezielte Untersuchung der Interaktion von Thrombin und Plättchen von großem Interesse.

Es ist daher wünschenswert, über Verfahren zu verfügen, die die Detektion von Modulatoren, also Inhibitoren oder Aktivatoren, der Plättchen-Thrombin-Interaktion in Patientenproben ermöglichen. Derartige Verfahren würden das Monitoring von Plättcheninhibitor-Therapien oder auch die Detektion von physiologischen Störfaktoren, wie z.B. aktivierenden oder inhibierenden Auto-Antikörpern ermöglichen.

Der vorliegenden Erfindung lag die Aufgabe zugrunde ein Verfahren zur gezielten Detektion von Modulatoren der GPIb-Thrombin-Interaktion in einer Probe bereit zu stellen.

Die Aufgabe wird dadurch gelöst, dass die Probe mit isoliertem GPIbα-Protein und mit isoliertem Thrombin in Kontakt gebracht wird und die Komplexbildung zwischen dem GPIbα-Protein und Thrombin bestimmt wird, wobei das GPIbα-Protein mutiert ist und verglichen mit der Wildtypsequenz des humanen GPIbα-Proteins mindestens die Aminosäurereste 1-268 enthält und an mindestens einer der Positionen 233, 235, 237 und 239 eine Substitution Xaa aufweist (SEQ ID NO: 1).

Der Begriff "Modulator der GPIb-Thrombin-Interaktion" umfasst Substanzen, die die GPIb-Thrombin-Interaktion beeinflussen. Inhibitoren der GPIb-Thrombin-Interaktion vermindern die Bindung von Thrombin an GPIbα-Protein. Aktivatoren der GPIb-Thrombin-Interaktion verstärken die Bindung von Thrombin an GPIbα-Protein.

Enthält die Probe einen Aktivator, ist die Komplexbildung gegenüber einer Normalprobe verstärkt.

Enthält die Probe einen Inhibitor, z.B.
- einen therapeutisch verabreichten Thrombininhibitor, z.B. aus der Gruppe der Exosite I-Inhibitoren (z.B. Hirudin) oder Exosite II-Inhibitoren (z.B. Heparin) (Ruggeri, Z.M. et al., Unravelling the mechanism and significance of thrombin binding to platelet glycoprotein Ib. Thrombosis and Haemostasis 2010, 104.5: 894-902) oder
- einen physiologischen Thrombininhibitor, wie z.B. Autoantikörper gegen Thrombin, die die Bindung von Thrombin an GPIbα verhindern, oder
- einen therapeutischen verabreichten GPIbα-Inhibitor, wie beispielsweise einen anti-GPIbα-Antikörper, z.B. den Antikörper 4H12 (US 5486361 A) oder den Antikörper SZ2 (Ruan, C. et al., A murine antiglycoprotein Ib complex monoclonal antibody, SZ 2, inhibits platelet aggregation induced by both ristocetin and collagen. Blood 1987, 69(2): 570-577) oder H6B4-Fab, das Fab-Fragment eines humanisierten monoklonalen anti-GPIbα-Antikörpers (Firbas, C. et al., Targeting von Willebrand factor and platelet glycoprotein Ib receptor. Expert Rev. Cardiovasc. Ther. 2010, 8(12): 1689-1701), oder GPG-290, einen rekombinanten, chimären Antikörper, der die aminoterminalen Aminosäuren 1-290 von GPIbα gekoppelt an humanes IgG1 enthält (Yeung, J. & Holinstat, M., Newer agents in antiplatelet therapy: a review. Journal of Blood Medicine 2012, 3: 33-42), oder
- einen physiologischen GPIbα-Inhibitor, wie z.B. Autoantikörper gegen GPIbα, die die Bindung von GPIbα an Thrombin verhindern, oder
- erhöhte Glycocalicin-Konzentrationen, die mit dem GPIbα-Protein um die Bindung an das Thrombin kompetieren,
ist die Komplexbildung gegenüber einer Normalprobe vermindert.

Gegenstand der vorliegenden Erfindung ist daher ein Verfahren zur Detektion von Modulatoren der GPIb-Thrombin-Interaktion in einer Probe, wobei die Probe mit isoliertem GPIbα-Protein und mit isoliertem Thrombin in Kontakt gebracht wird und die Komplexbildung zwischen dem GPIbα-Protein und Thrombin bestimmt wird. Das verwendete GPIbα-Protein ist mutiert und enthält verglichen mit der Wildtypsequenz des humanen GPIbα-Proteins mindestens die Aminosäurereste 1-268 und weist an mindestens einer der Positionen 233, 235, 237 und 239 eine Substitution Xaa (SEQ ID NO: 1) auf.

Vorteilhaft ist, dass dieses Verfahren ohne die Verwendung von Blutplättchen auskommt. Die Herstellung von Plättchen-Reagenzien aus tierischem oder menschlichem Blut ist aufwändig und garantiert keine konsistente Qualität.

Der Begriff "Probe" umfasst biologische Flüssigkeiten insbesondere von Menschen und Tieren, wie Blut, Plasma oder Serum.

Der Begriff "Normalprobe" umfasst ein Referenzmaterial, das bei Verwendung als Probe in dem erfindungsgemäßen Verfahren einen Messwert erzeugt, der der GPIb-Thrombin-Interaktion eines gesunden Individuums bzw. einer gesunden Population von Individuen entspricht, das bzw. die keine durch einen Modulator der GPIb-Thrombin-Interaktion beeinflusste GPIb-Thrombin-Interaktion aufweist. Als Referenzmaterial eignet sich beispielsweise ein Pool aus einer Körperflüssigkeit, beispielsweise ein Normalplasmapool oder Normalserumpool, von in der Regel mindestens 20 offensichtlich gesunden Individuen.

Bei dem in dem erfindungsgemäßen Verfahren verwendeten GPIbα-Protein kann es sich um ein rekombinant oder synthetisch hergestelltes GPIbα-Protein handeln. Zur Herstellung von rekombinantem GPIbα-Protein eignen sich bekannte prokaryontische oder eukaryontische Expressionssysteme, wie z.B. die Expression in Bakterien (z.B. E. coli), in Hefen (z.B. Saccharomyces cerevisiae, Pichia pastoris), in pflanzlichen, tierischen oder humanen Zellkulturen. Zur Herstellung von synthetischem GPIbα-Protein eignen sich bekannte Techniken zur in vitro-Proteinsynthese, wie z. B. Festphasensynthesen (z.B. Merrifield-Synthese). Bevorzugterweise handelt es sich bei dem in dem erfindungsgemäßen Verfahren verwendeten GPIbα-Protein um rekombinant hergestelltes GPIbα-Protein, das in einer Kultur humaner Zellen, bevorzugt in einer Kultur humaner embryonaler Nierenzellen (HEK-Zellen), hergestellt wurde.

Bevorzugterweise wird das GPIbα-Protein dem Testansatz in einer solchen Menge zugesetzt, dass eine Endkonzentration von 0,5-50 µg/mL GPIbα im Testansatz, besonders bevorzugt von 1-10 µg/mL GPIbα im Testansatz, ganz besonders bevorzugt von 5 µg/mL GPIbα im Testansatz erhalten wird.

Das in dem erfindungsgemäßen Verfahren verwendete GPIbα-Protein kann am N-Terminus mit der homologen humanen GPIbα-Signalsequenz MPLLLLLLLLPSPLHP (SEQ ID NO: 2, auch als Aminosäurereste -16 bis -1 bezeichnet) fusioniert sein. Alternativ kann das verwendete GPIbα-Protein am N-Terminus mit einer heterologen Signalsequenz fusioniert sein, d.h. mit einem Polypeptid, das üblicherweise nicht in dem humanen GPIbα-Polypeptid vorhanden ist, die aber in dem gewählten Expressionssystem die Expression und/oder Sekretion des rekombinant exprimierten GPIbα-Proteins positiv beeinflusst. Eine geeignete heterologe Signalsequenz ist z.B. MPLQLLLLLILLGPGNSLQLWDTWADEAEKALGPLLARDRR (SEQ ID NO: 3).

Weiterhin kann das in dem erfindungsgemäßen Verfahren verwendete GPIbα-Protein am C-Terminus mit einem oder mehreren Affinitäts-Tags fusioniert sein, die die Bindung des z.B. rekombinant exprimierten Proteins an einen Affinitätsträger ermöglichen, wodurch z.B. die Reinigung von rekombinant exprimiertem GPIbα-Protein ermöglicht wird. Bevorzugt sind kleine Affinitäts-Tags mit einer Länge von nicht mehr als 12 Aminosäuren. Besonders bevorzugt sind Affinitäts-Tags aus der Gruppe His-Tag, Flag-Tag, Arg-Tag, c-Myc-Tag und Strep-Tag. Geeignete Affinitätsträger, die mit hoher Affinität an ein Affinitäts-Tag binden, sind z.B. spezifische Antikörper, immobilisierte Kationen (z. B. Ni²⁺ mit Affinität für His-Tags) oder andere Typen von Bindungspartnern (z.B. Streptavidin mit Affinität für Strep-Tags).

Das verwendete isolierte GPIbα-Protein ist mutiert und enthält -verglichen mit der Wildtypsequenz des humanen GPIbα-Proteins (SEQ ID NO: 1)- mindestens die Aminosäurereste 1-268 und an mindestens einer der Positionen 233, 235, 237 und 239 eine Substitution Xaa. Bevorzugterweise enthält das mutierte GPIbα-Protein an zwei der Positionen 233, 235, 237 und 239 jeweils eine Substitution Xaa. Überraschenderweise wurde gefunden, dass die Verwendung von wildtypischem GPIbα-Protein nicht für die Detektion von Modulatoren der GPIb-Thrombin-Interaktion geeignet ist.

Bevorzugterweise bestehen die Substitutionen Xaa des Glycin-Rests an Position 233 und des Methionin-Rest an Position 239 der GPIbα-Kette aus einem Valin-Rest (G233V bzw. M239V) oder einem Serin-Rest (G233S bzw. M239S). Jede beliebige Kombination der unterschiedlichen Substitutionen Xaa an den beiden Positionen ist möglich. Besonders bevorzugt ist die Kombination G233V/M239V. Die Substitution Xaa des Asparaginsäurerestes an Position 235 besteht vorzugsweise aus einem Tyrosin-Rest (D235Y). Die Substitution Xaa des Lysin-Restes an Position 237 besteht vorzugsweise aus einem Valin-Rest (K237V). Bei den genannten Mutationen handelt es sich um gain-of-function Mutationen, die bekanntermaßen eine signifikant höhere Affinität für VWF aufweisen und stärker mit VWF interagieren als wildtypisches GPIbα-Protein. Dem Testansatz wird weder Ristocetin, Botrocetin noch eine Ristocetin-äquivalente Substanz hinzugefügt.

Bei dem in dem erfindungsgemäßen Verfahren verwendeten Thrombin kann es sich um rekombinantes oder aus natürlichen Quellen isoliertes humanes oder bovines Thrombin handeln.

In einer bevorzugten Ausführungsform des erfindungsgemäßen Verfahrens ist das Thrombin und/oder das GPIbα-Protein an eine Festphase assoziiert.

Der Begriff "assoziiert" ist breit zu verstehen und umfasst beispielsweise eine kovalente und eine nicht-kovalente Bindung, eine direkte und eine indirekte Bindung, die Adsorption an eine Oberfläche und den Einschluss in eine Vertiefung. Bei einer kovalenten Bindung ist das isolierte GPIbα-Protein über eine chemische Bindung an die Festphase gebunden. Ein Beispiel für eine nicht-kovalente Bindung ist die Oberflächenadsorption. Neben einer direkten Bindung an die Festphase kann das isolierte GPIbα-Protein oder das Thrombin auch indirekt über spezifische Wechselwirkung mit anderen spezifischen Bindungspartnern an die Festphase gebunden sein, z. B. über spezifische Wechselwirkung mit einem Antikörper oder einem Antikörperfragment, bevorzugt mit einem anti-GPIbα- beziehungsweise mit einem anti-Thrombin-Antikörper oder -sofern das isolierte Protein über ein Affinitäts-Tag verfügt- mit einem anti-Affinitäts-Tag-Antikörper.

Der Begriff "Festphase" im Sinne dieser Erfindung beinhaltet einen Gegenstand, der aus porösem und/oder nicht porösem, wasserunlöslichem Material besteht und die unterschiedlichsten Formen aufweisen kann, wie z.B. Gefäß, Röhrchen, Mikrotitrationsplatte (ELISA-Platte), Kugel, Mikropartikel, Stäbchen, Streifen, Filter- oder Chromatographiepapier, etc. In der Regel ist die Oberfläche der Festphase hydrophil oder kann hydrophil gemacht werden. Die Festphase kann aus den unterschiedlichsten Materialien bestehen wie z.B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere wie z.B. Cellulose, Nitrocellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Latex; Keramik; Glas; Metalle, insbesondere Edelmetalle wie Gold und Silber; Magnetit; Mischungen oder Kombinationen derselben. Der Begriff "Festphase" umfasst explizit nicht Zellen, insbesondere nicht Plättchen (Thromobozyten). Es handelt sich also in jedem Fall um eine nicht-thrombozytäre Festphase.

Die Festphase kann einen Überzug aus einer oder mehreren Schichten aufweisen, z.B. aus Proteinen, Kohlehydraten, lipophilen Substanzen, Biopolymeren, organischen Polymeren oder Mischungen hiervon, um beispielsweise die unspezifische Bindung von Probenbestandteilen an die Festphase zu unterdrücken oder zu verhindern oder um beispielsweise Verbesserungen zu erreichen hinsichtlich der Suspensionsstabilität von partikulären Festphasen, der Lagerstabilität, der formgebenden Stabilität oder der Resistenz gegen UV-Licht, Mikroben oder sonstige zerstörend wirkender Agenzien.

Durch das Inkontaktbringen von isoliertem GPIbα-Protein mit Thrombin bildet sich ein Komplex aus den beiden Komponenten. Sind in der zugegebenen Patientenprobe Substanzen enthalten, die diese Komplexbildung beeinflussen, z.B. GPIb- oder Thrombin-Inhibitoren oder -Aktivatoren, wird eine gegenüber dem Normal veränderte Komplexbildung gemessen. Das Normal wird durch die Bestimmung der GPIb-Thrombin-Interaktion in geeigneten Referenzmaterialien, beispielsweise in einem Normalplasmapool, bestimmt und kann beispielsweise als 100 % der Norm festgelegt werden. Die GPIb-Thrombin-Interaktion, die in einer Probe eines Individuums bestimmt wird, kann dann zu dem Referenzwert in Relation gesetzt werden.

In einer Ausführungsform des erfindungsgemäßen Verfahrens ist mindestens eine der beiden Komponenten, also GPIbα und/oder Thrombin, mit einer partikulären Festphase, bevorzugt mit Latexpartikeln assoziiert. Die Komplexbildung zwischen Thrombin, GPIbα-Protein und der/den assoziierten Festphase(n) kann dann durch Messung der Agglutination der partikulären Festphase bestimmt werden. Zur quantitativen Bestimmung der Agglutinationsreaktion, die mit der Komplexbildung korreliert, kann z.B. die Lichtstreuung an den Partikelaggregaten über die Messung der Streulichintensität (Nephelometrie) oder über die Messung der Trübung des Mediums (Turbidimetrie) genutzt werden.

In einer anderen Ausführungsform des erfindungsgemäßen Verfahrens ist bzw. wird jede der beiden Komponenten, also GPIbα und Thrombin, mit einer ersten und einer zweiten Komponente eines signalbildenden Systems assoziiert, welche so zusammenwirken, dass ein detektierbares Signal entsteht, wenn die erste und die zweite Komponente des signalbildenden Systems in räumliche Nähe zueinander gebracht werden. Unter einem Zusammenwirken zwischen den Komponenten ist insbesondere ein Energietransfer - also die direkte Übertragung von Energie zwischen den Komponenten, z.B. durch Licht- oder Elektronenstrahlung sowie über reaktive chemische Moleküle, wie z.B. kurzlebigen Singulett-Sauerstoff - zu verstehen. Der Energietransfer kann von einer auf eine andere Komponente erfolgen, möglich ist aber auch eine Kaskade verschiedener Substanzen, über die der Energietransfer läuft.

Zum Beispiel kann es sich bei den Komponenten um ein Paar aus einem Energiespender und einem Energieempfänger handeln, wie beispielsweise Photosensitizer und chemilumineszierendes Agens (EP-A2-0515194, LOCI® Technologie) oder Photosensitizer und Fluorophor (WO-A1-95/06877) oder radioaktives Iod<125> und Fluorophor, oder Fluorophor und Fluoreszenz-Quencher. In einer anderen Ausführungsform des erfindungsgemäßen Verfahrens ist das Thrombin mit einer nicht-partikulären Festphase assoziiert, bevorzugterweise mit der Oberfläche einer Mikrotiterplatte. Die Komplexbildung zwischen Thrombin und GPIbα-Protein kann dann durch Messung der GPIbα-Menge, die über das Thrombin an die Festphase gebunden wird, bestimmt werden. Zur Bestimmung der GPIbα-Menge, die über das Thrombin an die Festphase gebunden wurde, kann beispielweise ein anti-GPIbα-Antikörper verwendet werden, der direkt oder indirekt mit einer Komponente eines signalbildenden Systems assoziiert ist, und damit die Quantifizierung der gebundenen GPIbα-Menge erlaubt. Alternativ kann das GPIbα-Protein mit einer nicht-partikulären Festphase assoziiert sein, und die Komplexbildung zwischen Thrombin und GPIbα-Protein durch Messung der Thrombin-Menge, die über das GPIbα-Protein an die Festphase gebunden wird, bestimmt werden. Zur Bestimmung der Thrombin-Menge, die über das GPIbα an die Festphase gebunden wurde, kann beispielweise ein anti-Thrombin-Antikörper verwendet werden, der direkt oder indirekt mit einer Komponente eines signalbildenden Systems assoziiert ist oder ein Peptidsubstrat mit einer durch Thrombin abspaltbaren Signalgruppe, beispielsweise einer chromogenen, fluorogenen oder elektrogenen Signalgruppe.

Ein weiterer Gegenstand der vorliegenden Erfindung ist ein Testkit zur Durchführung eines erfindungsgemäßen Verfahrens enthaltend ein erstes Reagenz, das isoliertes GPIbα-Protein enthält, wobei das GPIbα-Protein mutiert ist und verglichen mit der Wildtypsequenz des humanen GPIbα-Proteins mindestens die Aminosäurereste 1-268 enthält und an mindestens einer der Positionen 233, 235, 237 und 239 eine Substitution Xaa aufweist (SEQ ID NO: 1) und ein zweites Reagenz, das Thrombin enthält. Besonders bevorzugt ist ein Testkit enthaltend ein Reagenz, das isoliertes, mutiertes GPIbα-Protein enthält, das an mindestens zwei der Positionen 233, 235, 237 und 239 jeweils eine Substitution Xaa aufweist, besonders bevorzugt an den Positionen 233 und 239. Ganz besonders bevorzugt bestehen die Substitutionen Xaa des Glycin-Rests an Position 233 und des Methionin-Rest an Position 239 der GPIbα-Kette aus einem Valin-Rest (G233V bzw. M239V). Ein anderes bevorzugtes Testkit enthält ein Reagenz, das isoliertes, mutiertes GPIbα-Protein enthält, das an den Positionen 233, 235 und 239 jeweils eine Substitution Xaa aufweist. Bevorzugterweise bestehen die Substitutionen Xaa des Glycin-Rests an Position 233 und des Methionin-Rest an Position 239 der GPIbα-Kette aus einem Valin-Rest (G233V bzw. M239V) oder einem Serin-Rest (G233S bzw. M239S) und die Substitution Xaa des Asparaginsäurerestes an Position 235 aus einem Tyrosin-Rest (D235Y).

In einer Ausführungsform des Testkits kann das zweite Reagenz eine Festphase umfassen, an die das Thrombin assoziiert ist. Bevorzugterweise enthält ein solches Testkit ferner ein weiteres oder mehrere weitere Reagenzien zum Nachweis des isolierten GPIbα-Proteins, enthaltend beispielsweise einen anti-GPIbα-Antikörper oder einen anti-Tag-Antikörper, der direkt oder indirekt mit einem Enzym markiert ist und ein Substrat für das Enzym, beispielsweise einen Meerrettichperoxidase-markierten Antikörper und Tetramethylbenzidin als chromogenes Substrat.

In einer anderen Ausführungsform des Testkits kann das erste Reagenz eine Festphase umfassen, an die das mutierte GPIbα-Protein assoziiert ist. Bevorzugterweise enthält ein solches Testkit ferner ein weiteres oder mehrere weitere Reagenzien zum Nachweis von Thrombin, enthaltend beispielsweise einen anti-Thrombin-Antikörper der direkt oder indirekt mit einem Enzym markiert ist und ein Substrat für das Enzym, beispielsweise einen Meerrettichperoxidase-markierten Antikörper und Tetramethylbenzidin als chromogenes Substrat. Alternativ kann zum Nachweis von Thrombin auch ein Peptidsubstrat mit einer durch Thrombin abspaltbaren Signalgruppe verwendet werden.

Die Reagenzien können in flüssiger oder lyophilisierter Form bereitgestellt werden. Für den Fall, dass ein Reagenz als Lyophilisat vorliegt, kann das Testkit zusätzlich ein zur Suspendierung des Lyophilisats erforderliches Lösemittel enthalten, wie z.B. destilliertes Wasser oder einen geeigneten Puffer.

### Figurenbeschreibung

### Figur 1

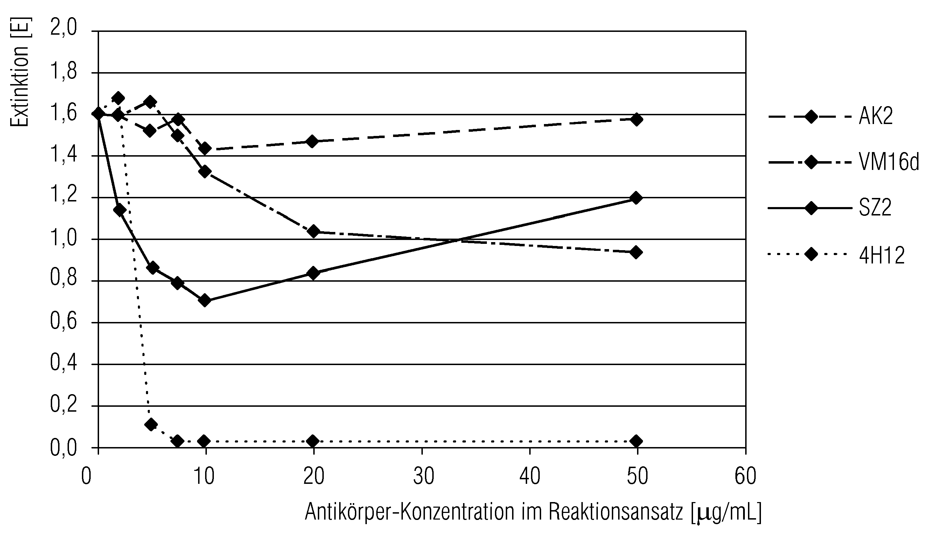

Figur 1 zeigt die Extinktionsmesswerte [E] von Reaktionsansätzen mit unterschiedlichen anti-GPIb-Antikörpern in verschiedenen Konzentrationen. Die Antikörper VM16d, SZ2 und 4H12 inhibieren die Thrombin-GPIb-Interaktion in konzentrationsabhängiger Weise (siehe Beispiel 1).

### BEISPIELE

### BEISPIEL 1: Detektion von inhibitorischen GPIb-Antikörpern in einer Probe

Eine Mikrotiter-Platte wurde mit Antikörpern gegen humanes Thrombin beschichtet.Humanes Thrombin wurde von Sigma-Aldrich bezogen (T7009, Sigma-Aldrich, Hamburg, Deutschland). Pro Vertiefung wurden 100 µL einer Lösung von 1 µg/mL Thrombin in Glycerin-Puffer (8 mL A. dest., 87,7 mg NaCl, 69 mg NaH₂PO₄*H₂O, 813 µL 87 %iges Glycerin, 18 µL Tween® 20, 10 mg Rinderalbumin, 1 mg Rinder-IgG, 2 mg Phenol, 18,6 mg Titriplex I, mit ca. 43 µL 10 N NaOH auf pH 6,8 eingestellt) zugegeben und bei Raumtemperatur eine Stunde inkubiert. Danach erfolgte ein viermaliges Waschen mit 300 µL Waschpuffer.

Es wurde ein rekombinant hergestelltes, Flag-Tag-fusioniertes GPIbα-Protein verwendet (aa 1-268), bei dem der Glycin-Rest an Position 233 und der Methionin-Rest an Position 239 jeweils durch einen Valin-Rest ersetzt ist (G233V, M239V). Von diesem GPIbα-Protein wurden jeweils 200 µL einer 10 µg/mL-Lösung in Glycerin-Puffer mit 200 µL der inhibitorischen anti-GPIbα-Antikörper 4H12, SZ2 und VM16D bzw. des Kontrollantikörpers AK2 in verschiedenen Konzentrationen in Glycerin-Puffer gemischt und eine Stunde und 10 Minuten bei Raumtemperatur inkubiert. Von diesen GPIbα-Protein/Antikörper-Mischungen wurden jeweils 100 µL in eine Vertiefung der Mikrotiter-Platte pipettiert und eine Stunde bei Raumtemperatur inkubiert. Danach erfolgte ein viermaliges Waschen mit 300 µL Waschpuffer.

Zum quantitativen Nachweis des gebundenen, Flag-Tagfusionierten GPIbα-Proteins wurden jeweils 100 µL einer 0,06 µg/mL Lösung der Anti-Flag M2-Peroxidase (Sigma-Aldrich, Hamburg, Deutschland) in Glycerin-Puffer pro Vertiefung zugegeben und eine Stunde bei Raumtemperatur inkubiert. Nach viermaligem Waschen mit 300 µL Waschpuffer wurden 100 µL einer Lösung des chromogenen Peroxidasesubstrats TMB (Tetramethylbenzidin-dihydrochlorid) und Wasserstoffperoxid pro Vertiefung zugegeben und für 20 Minuten inkubiert. Die Reaktion wurde abgestoppt, und die Extinktion der Reaktionsansätze wurde mit Licht mit einer Wellenlänge von 450 nm in einem ELISA-Platten Reader unter Verwendung einer Referenzwellenlänge von 650 nm gemessen.

Die Extinktionsmesswerte sind in Figur 1 dargestellt.

Der anti-GPIb-Antikörper AK2 wurde als Kontroll-Antikörper eingesetzt, welcher bekanntermaßen nicht die Bindung von Thrombin an das GPIbα-Protein beeinflusst, sondern die Ristocetin-induzierte Bindung von VWF an das GPIbα-Protein hemmt (Ward, C.M. et al., Mocarhagin, a novel cobra venom metalloproteinase, cleaves the platelet von Willebrand factor receptor glycoprotein Ibα. Identification of the sulfated tyrosine/anionic sequence Tyr-276-Glu-282 of glycoprotein Ibα as a binding site for von Willebrand factor and α-thrombin. Biochemistry 1996, 35: 4929-4938).

Der anti-GPIb-Antikörper 4H12 hemmt die Bindung von Thrombin an das GPIbα-Protein bekanntermaßen sehr stark (Gralnick, US 5486361 A).

Der anti-GPIb-Antikörper SZ2 (Ruan, C. et al., 1987) ist als inhibierendes Anti-Plättchen-Therapeutikum in Entwicklung (Yeung, J. & Holinstat, M., 2012).

Der anti-GPIb-Antikörper VM16d ist ebenfalls bekannt als Antikörper, welcher die Thrombin-Bindung an GPIb hemmt (Dubois, C. et al., Thrombin binding to GPIbα induces integrin αIibβ3 dependent platelet adhesion to fibrin in ex vivo flowing whole blood. Thromb Haemost 2004, 91: 233-237).

Wie aus Figur 1 hervorgeht, hemmen 4H12, SZ2 und VM16d im erfindungsgemäßen Verfahren die GPIbα-Thrombin-Interaktion in konzentrationsabhängiger Weise. Das Verfahren eignet sich daher zur Detektion von Inhibitoren der GPIb-Thrombin-Interaktion in einer Probe.

## Patentansprüche

1. Verfahren zur Detektion von Modulatoren der GPIb-Thrombin-Interaktion in einer Probe, wobei die Probe mit isoliertem GPIbα-Protein und mit isoliertem Thrombin in Kontakt gebracht wird und die Komplexbildung zwischen dem GPIbα-Protein und Thrombin bestimmt wird, **dadurch gekennzeichnet, dass** das GPIbα-Protein mutiert ist und verglichen mit der Wildtypsequenz des humanen GPIbα-Proteins mindestens die Aminosäurereste 1-268 enthält und an mindestens einer der Positionen 233, 235, 237 und 239 eine Substitution Xaa aufweist (SEQ ID NO: 1).

2. Verfahren nach Anspruch 1, wobei das mutierte GPIbα-Protein mindestens eine Substitution aus der Gruppe G233V, G233S, D235Y, D235V, K237V, M239V und M239S aufweist.

3. Verfahren nach einem der vorherigen Ansprüche, wobei die Probe nicht mit Ristocetin oder Botrocetin in Kontakt gebracht wird.

4. Verfahren nach einem der vorherigen Ansprüche, wobei das Thrombin und/oder das GPIbα-Protein an eine Festphase assoziiert ist.

5. Verfahren nach einem der vorherigen Ansprüche, wobei das Thrombin und/oder das GPIbα-Protein über einen Bindungspartner, vorzugsweise über einen Antikörper an die Festphase assoziiert ist.

6. Verfahren nach Anspruch 4, wobei die Festphase eine partikuläre Festphase ist.

7. Verfahren nach Anspruch 6, wobei die Komplexbildung zwischen Thrombin und GPIbα-Protein anhand der Agglutination der partikulären Festphase bestimmt wird.

8. Verfahren nach Anspruch 4, wobei die Festphase eine nicht-partikuläre Festphase ist, bevorzugt die Oberfläche einer Mikrotitrationsplatte.

9. Testkit zur Durchführung eines Verfahrens gemäß Anspruch 1 enthaltend ein erstes Reagenz, das isoliertes GPIbα-Protein enthält, wobei das GPIbα-Protein mutiert ist und verglichen mit der Wildtypsequenz des humanen GPIbα-Proteins mindestens die Aminosäurereste 1-268 enthält und an mindestens einer der Positionen 233, 235, 237 und 239 eine Substitution Xaa aufweist (SEQ ID NO: 1), und ein zweites Reagenz, das Thrombin enthält.

10. Testkit nach Anspruch 9, wobei das zweite Reagenz eine Festphase umfasst, an die das Thrombin assoziiert ist.

11. Testkit nach einem der Ansprüche 9 und 10, ferner enthaltend ein oder mehrere Reagenzien zum Nachweis des isolierten GPIbα-Proteins.

12. Testkit nach Anspruch 11, wobei das oder die Reagenzien zum Nachweis des isolierten GPIbα-Proteins einen Enzym-markierten Antikörper mit Spezifität für das isolierte GPIbα-Protein und ein Substrat für das Enzym umfassen.

13. Testkit nach Anspruch 9, wobei das erste Reagenz eine Festphase umfasst, an die das isolierte GPIbα-Protein assoziiert ist.

14. Testkit nach einem der Ansprüche 9 und 13, ferner enthaltend ein oder mehrere Reagenzien zum Nachweis von Thrombin.

15. Testkit nach Anspruch 14, wobei das oder die Reagenzien zum Nachweis von Thrombin entweder einen Enzym-markierten Antikörper mit Spezifität für Thrombin und ein Substrat für das Enzym umfassen oder ein Peptidsubstrat mit einer durch Thrombin abspaltbaren Signalgruppe umfassen.

## Claims

1. Method for detecting modulators of GPIb-thrombin interaction in a sample, the sample being contacted with isolated GPIbα protein and with isolated thrombin and the formation of a complex between the GPIbα protein and thrombin being determined, **characterized in that** the GPIbα protein is mutated and, compared to the wild-type sequence of the human GPIbα protein, contains at least the amino acid residues 1-268 and has a substitution Xaa at at least one of the positions 233, 235, 237 and 239 (SEQ ID NO: 1).

2. Method according to Claim 1, wherein the mutated GPIbα protein has at least one substitution from the group consisting of G233V, G233S, D235Y, D235V, K237V, M239V and M239S.

3. Method according to either of the preceding claims, wherein the sample is not contacted with ristocetin or botrocetin.

4. Method according to any of the preceding claims, wherein the thrombin and/or the GPIbα protein is associated with a solid phase.

5. Method according to any of the preceding claims, wherein the thrombin and/or the GPIbα protein is associated with the solid phase via a binding partner, preferably via an antibody.

6. Method according to Claim 4, wherein the solid phase is a particulate solid phase.

7. Method according to Claim 6, wherein the formation of a complex between thrombin and GPIbα protein is determined on the basis of the agglutination of the particulate solid phase.

8. Method according to Claim 4, wherein the solid phase is a nonparticulate solid phase, preferably the surface of a microtitration plate.

9. Assay kit for carrying out a method according to Claim 1, containing a first reagent containing isolated GPIbα protein, the GPIbα protein being mutated and, compared to the wild-type sequence of the human GPIbα protein, containing at least the amino acid residues 1-268 and having a substitution Xaa at at least one of the positions 233, 235, 237 and 239 (SEQ ID NO: 1), and a second reagent containing thrombin.

10. Assay kit according to Claim 9, wherein the second reagent comprises a solid phase to which the thrombin is associated.

11. Assay kit according to either of Claims 9 and 10, further containing one or more reagents for detecting the isolated GPIbα protein.

12. Assay kit according to Claim 11, wherein the reagent(s) for detecting the isolated GPIbα protein comprise(s) an enzyme-labeled antibody specific for the isolated GPIbα protein and a substrate for the enzyme.

13. Assay kit according to Claim 9, wherein the first reagent comprises a solid phase to which the isolated GPIbα protein is associated.

14. Assay kit according to either of Claims 9 and 13, further containing one or more reagents for detecting thrombin.

15. Assay kit according to Claim 14, wherein the reagent(s) for detecting thrombin comprise(s) either an enzyme-labeled antibody specific for thrombine and a substrate for the enzyme or a peptide substrate having a thrombin-cleavable signal group.

## Revendications

1. Procédé de détection de modulateurs de l'interaction GPIb-thrombine dans un échantillon, en mettant l'échantillon en contact avec de la GPIbα-protéine isolée et avec de la thrombine isolée et en déterminant la formation de complexe entre la GPIbα-protéine et la thrombine, **caractérisé en ce que** la GPIbα-protéine est mutée et, par rapport à la séquence de type sauvage de la GPIbα-protéine humaine, contient au moins les restes d'acides aminés 1 à 268 et a une substitution Xaa sur au moins l'une des positions 233, 235, 237 et 239 ( identification de séquence n°1 ).

2. Procédé suivant la revendication 1, dans lequel la GPIbα-protéine mutée a au moins une substitution choisie dans le groupe consistant en G233V, G233S, D235V, K237V, M239V et M239S.

3. Procédé suivant l'une des revendications précédentes, dans lequel on ne met pas l'échantillon en contact avec de la ristocétine ou de la botrocétine.

4. Procédé suivant l'une des revendications précédentes, dans lequel la thrombine et/ou la GPIbα-protéine est associée à une phase solide.

5. Procédé suivant l'une des revendications précédentes, dans lequel la thrombine et/ou la GPIbα-protéine est associée à la phase solide par l'intermédiaire d'un partenaire de fixation, de préférence par l'intermédiaire d'un anticorps.

6. Procédé suivant la revendication 4, dans lequel la phase solide est une phase solide particulaire.

7. Procédé suivant la revendication 6, dans lequel on détermine la formation d'un complexe entre la thrombine et la GPIbα-protéine à l'aide de l'agglutination de la phase solide particulaire.

8. Procédé suivant la revendication 4, dans lequel la phase solide est une phase solide non particulaire, de préférence la surface d'une plaquette de microtitration.

9. Trousse de test pour effectuer un procédé suivant la revendication 1, contenant un premier réactif qui contient la GPIbα-protéine isolée, la GPIbα-protéine étant mutée et contenant, par rapport à la séquence de type sauvage de la GPIbα-protéine humaine, au moins les restes d'acides aminés 1 à 268 et ayant une substitution Xaa sur au moins l'une des positions 233, 235, 237 et 239 ( identification de séquence n° 1 ), et un deuxième réactif qui contient de la thrombine.

10. Trousse de test suivant la revendication 9, dans laquelle le deuxième réactif comprend une phase solide à laquelle la thrombine est associée.

11. Trousse de test suivant l'une des revendications 9 et 10, contenant en outre un ou plusieurs réactifs de détection de la GPIbα-protéine isolée.

12. Trousse de test suivant la revendication 11, dans laquelle le ou les réactifs de détection de la GPIbα-protéine isolée comprennent un anticorps marqué par un enzyme et ayant de la spécificité pour la GPIbα-protéine isolée et un substrat pour l'enzyme.

13. Trousse de test suivant la revendication 9, dans laquelle le premier réactif comprend une phase solide à laquelle la GPIbα-protéine isolée est associée.

14. Trousse de test suivant l'une des revendications 9 et 13, contenant en outre un ou plusieurs réactifs de détection de la thrombine.

15. Trousse de test suivant la revendication 14, dans laquelle le ou les réactifs de détection de la thrombine comprennent ou bien un anticorps marqué par un enzyme et ayant de la spécificité pour la thrombine et un substrat pour l'enzyme, ou bien un substrat de peptide ayant un groupe de signal clivable par de la thrombine.


### SEQUENCE LISTING

<110> Siemens Healthcare Diagnostics Products GmbH
<120> Verfahren zur Bestimmung von Modulatoren der GPIb-Thrombin-Interaktion
<130> 2013P22440EP
<160> 3
<170> PatentIn version 3.5
<210> 1
   <211> 610
   <212> PRT
   <213> Homo sapiens
<400> 1
   His Pro Ile Cys Glu Val Ser Lys Val Ala Ser His Leu Glu Val Asn
   1               5                   10                  15
   Cys Asp Lys Arg Asn Leu Thr Ala Leu Pro Pro Asp Leu Pro Lys Asp
               20                  25                  30
   Thr Thr Ile Leu His Leu Ser Glu Asn Leu Leu Tyr Thr Phe Ser Leu
           35                  40                  45
   Ala Thr Leu Met Pro Tyr Thr Arg Leu Thr Gln Leu Asn Leu Asp Arg
       50                  55                  60
   Cys Glu Leu Thr Lys Leu Gln Val Asp Gly Thr Leu Pro Val Leu Gly
   65                  70                  75                  80
   Thr Leu Asp Leu Ser His Asn Gln Leu Gln Ser Leu Pro Leu Leu Gly
                   85                  90                  95
   Gln Thr Leu Pro Ala Leu Thr Val Leu Asp Val Ser Phe Asn Arg Leu
               100                 105                 110
   Thr Ser Leu Pro Leu Gly Ala Leu Arg Gly Leu Gly Glu Leu Gln Glu
           115                 120                 125
   Leu Tyr Leu Lys Gly Asn Glu Leu Lys Thr Leu Pro Pro Gly Leu Leu
       130                 135                 140
   Thr Pro Thr Pro Lys Leu Glu Lys Leu Ser Leu Ala Asn Asn Asn Leu
   145                 150                 155                 160
   Thr Glu Leu Pro Ala Gly Leu Leu Asn Gly Leu Glu Asn Leu Asp Thr
                   165                 170                 175
   Leu Leu Leu Gln Glu Asn Ser Leu Tyr Thr Ile Pro Lys Gly Phe Phe
               180                 185                 190
   Gly Ser His Leu Leu Pro Phe Ala Phe Leu His Gly Asn Pro Trp Leu
           195                 200                 205
   Cys Asn Cys Glu Ile Leu Tyr Phe Arg Arg Trp Leu Gln Asp Asn Ala
       210                 215                 220
   Glu Asn Val Tyr Val Trp Lys Gln Gly Val Asp Val Lys Ala Met Thr
   225                 230                 235                 240
   Ser Asn Val Ala Ser Val Gln Cys Asp Asn Ser Asp Lys Phe Pro Val
                   245                 250                 255
   Tyr Lys Tyr Pro Gly Lys Gly Cys Pro Thr Leu Gly Asp Glu Gly Asp
               260                 265                 270
   Thr Asp Leu Tyr Asp Tyr Tyr Pro Glu Glu Asp Thr Glu Gly Asp Lys
           275                 280                 285
   Val Arg Ala Thr Arg Thr Val Val Lys Phe Pro Thr Lys Ala His Thr
       290                 295                 300
   Thr Pro Trp Gly Leu Phe Tyr Ser Trp Ser Thr Ala Ser Leu Asp Ser
   305                 310                 315                 320
   Gln Met Pro Ser Ser Leu His Pro Thr Gln Glu Ser Thr Lys Glu Gln
                   325                 330                 335
   Thr Thr Phe Pro Pro Arg Trp Thr Pro Asn Phe Thr Leu His Met Glu
               340                 345                 350
   Ser Ile Thr Phe Ser Lys Thr Pro Lys Ser Thr Thr Glu Pro Thr Pro
           355                 360                 365
   Ser Pro Thr Thr Ser Glu Pro Val Pro Glu Pro Ala Pro Asn Met Thr
       370                 375                 380
   Thr Leu Glu Pro Thr Pro Ser Pro Thr Thr Pro Glu Pro Thr Ser Glu
   385                 390                 395                 400
   Pro Ala Pro Ser Pro Thr Thr Pro Glu Pro Thr Pro Ile Pro Thr Ile
                   405                 410                 415
   Ala Thr Ser Pro Thr Ile Leu Val Ser Ala Thr Ser Leu Ile Thr Pro
               420                 425                 430
   Lys Ser Thr Phe Leu Thr Thr Thr Lys Pro Val Ser Leu Leu Glu Ser
           435                 440                 445
   Thr Lys Lys Thr Ile Pro Glu Leu Asp Gln Pro Pro Lys Leu Arg Gly
       450                 455                 460
   Val Leu Gln Gly His Leu Glu Ser Ser Arg Asn Asp Pro Phe Leu His
   465                 470                 475                 480
   Pro Asp Phe Cys Cys Leu Leu Pro Leu Gly Phe Tyr Val Leu Gly Leu
                   485                 490                 495
   Phe Trp Leu Leu Phe Ala Ser Val Val Leu Ile Leu Leu Leu Ser Trp
               500                 505                 510
   Val Gly His Val Lys Pro Gln Ala Leu Asp Ser Gly Gln Gly Ala Ala
           515                 520                 525
   Leu Thr Thr Ala Thr Gln Thr Thr His Leu Glu Leu Gln Arg Gly Arg
       530                 535                 540
   Gln Val Thr Val Pro Arg Ala Trp Leu Leu Phe Leu Arg Gly Ser Leu
   545                 550                 555                 560
   Pro Thr Phe Arg Ser Ser Leu Phe Leu Trp Val Arg Pro Asn Gly Arg
                   565                 570                 575
   Val Gly Pro Leu Val Ala Gly Arg Arg Pro Ser Ala Leu Ser Gln Gly
               580                 585                 590
   Arg Gly Gln Asp Leu Leu Ser Thr Val Ser Ile Arg Tyr Ser Gly His
           595                 600                 605
   Ser Leu
       610
<210> 2
   <211> 16
   <212> PRT
   <213> Homo sapiens
<400> 2
   Met Pro Leu Leu Leu Leu Leu Leu Leu Leu Pro Ser Pro Leu His Pro
   1               5                   10                  15
<210> 3
   <211> 41
   <212> PRT
   <213> Artificial
<220>
   <223> heterologous signal peptide
<400> 3
   Met Pro Leu Gln Leu Leu Leu Leu Leu Ile Leu Leu Gly Pro Gly Asn
   1               5                   10                  15
   Ser Leu Gln Leu Trp Asp Thr Trp Ala Asp Glu Ala Glu Lys Ala Leu
               20                  25                  30
   Gly Pro Leu Leu Ala Arg Asp Arg Arg
           35                  40